# EUROPEAN PATENT APPLICATION

(11) **EP 4 647 072 A1**
(43) Date of publication of application: **12.11.2025**
(21) Application number: 24766967.4
(22) Date of filing: 27.02.2024
(51) Int. Cl.: A61K 31/385, A61K 38/06, A61K 38/19, A61P 43/00

(54) **ENHANCER OF PHYSIOLOGICAL ACTIVITY OF HEPATOCYTE GROWTH FACTOR**

(30) Priority: 07.03.2023 JP 2023034610
(71) Applicant: Kyowa Hakko Bio Co., Ltd., Tokyo 100-8185 (JP); Kyushu University, National University Corporation, Nishi-ku Fukuoka-shi Fukuoka 819-0395 (JP)
(72) Inventor: OHSHIMA Etsuo, Tokyo 100-8185 (JP); TATSUMI Ryuichi, Fukuoka-shi, Fukuoka 819-0395 (JP); NAKAMURA Mako, Fukuoka-shi, Fukuoka 819-0395 (JP)
(74) Representative: Vossius & Partner Patentanwälte Rechtsanwälte mbB
(86) International application number: PCT/JP2024/007127
(87) International publication number: WO 2024/185596

(57) **Abstract**

Disclosed is an enhancer of the physiological activity of a hepatocyte growth factor, the enhancer comprising a trisulfide compound. The trisulfide compound comprises at least one component selected from the group consisting of glutathione trisulfide, a pharmaceutically acceptable salt of glutathione trisulfide, a compound represented by formula (1), a pharmaceutically acceptable salt of the compound, a cyclodextrin inclusion complex of the compound or the pharmaceutically acceptable salt, a compound represented by formula (6), a pharmaceutically acceptable salt of the compound, and a cyclodextrin inclusion complex of the compound or the pharmaceutically acceptable salt.

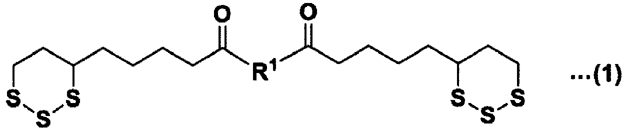

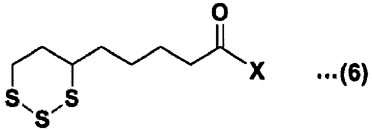

## Description

### Technical Field

The present invention relates to an enhancer of physiological activity of hepatocyte growth factor.

### Background Art

Hepatocyte Growth Factor (hereinafter also referred to as "HGF") was isolated and purified from the plasma of patients with fulminant hepatitis in the 1980s as a cytokine that promotes the growth of primary cultured hepatocytes, and subsequently its cDNA was cloned. HGF is secreted as an inactive single-chain form (pro-HGF) and is cleaved by proteases to the active form of HGF, a heterodimer consisting of a light chain and a heavy chain, both chains being linked by a disulfide bond. Subsequent research has revealed that HGF has diverse functions responsible for tissue and organ regeneration and repair, such as promoting cell proliferation, cell migration, inhibiting cell death, anti-fibrosis, and angiogenesis. Research and development for therapeutic applications has been conducted for various diseases including liver cirrhosis, chronic renal failure, pulmonary fibrosis, myocardial infarction, arteriosclerosis obliterans, amyotrophic lateral sclerosis, acute phase of spinal cord injury and the like. The diverse biological effects of HGF are exerted through binding to c-Met, which is the product of the oncogene c-Met and has tyrosine kinase activity (Non-Patent Literatures 1 and 2). HGF has been reported to be an activating factor for muscle stem cells (satellite cells), which are known to contribute to muscle fiber regeneration. This action is also exerted through c-Met (Non-Patent Literature 3).

Trisulfide compounds such as glutathione trisulfide are converted to reactive sulfur species such as glutathione persulfide *in vivo.* Reactive sulfur species have been reported to possess strong antioxidant effects and potentially diverse physiological functions such as anti-aging (see, for example, Non-Patent Literatures 4 to 7). Methods for producing trisulfide compounds are known, such as those described in Patent Literatures 1 and 2.

### Citation List

### Patent Literature

[Patent Literature 1] WO 2018/117186
[Patent Literature 2] WO 2022/045212

### Non Patent Literature

[Non-Patent Literature 1] Eiichi Gohda, "Function and Production Control of Hepatocyte Growth Factor (HGF)", Folia Pharmacol. Jpn. 119, 287-294 (2002)
[Non-Patent Literature 2] Shinya Mizuno et al., "Clinical Application of Regeneration Factor HGF", Drug Delivery System 16-6, 473-484 (2001)
[Non-Patent Literature 3] R. Tatsumi, "Mechano-biology of skeletal muscle hypertrophy and regeneration: possible mechanism of stretch-induced activation of resident myogenic stem cells", Animal Science Journal 81(1), 11-20 (2010).
[Non-Patent Literature 4] T. Ida, et al., "Reactive cysteine persulfides and S-polythiolation regulate oxidative stress and redox signaling", PNAS, May 27, 2014, 111(21) 7606-7611
[Non-Patent Literature 5] T. Akaike, et al., "Cysteinyl-tRNA synthetase governs cysteine polysulfidation and mitochondrial bioenergetics", Nature Communications, 2017, Oct 27; 8(1): 1177
[Non-Patent Literature 6] M. Ezaka, et al., "Oral Administration of Glutathione Trisulfide Increases Reactive Sulfur Levels in Dorsal Root Ganglion and Ameliorates Paclitaxel-Induced Peripheral Neuropathy in Mice", Antioxidants 11, 2122 (2022)
[Non-Patent Literature 7] F. Nagashima, et al., "Sulfide: quinone oxidoreductase ameliorates neurodegeneration in a murine model of Parkinson's disease", Redox Biol. Feb; 59: 102562. (2023)

### Summary of Invention

### Technical Problem

An object of the present invention is to provide an enhancer of physiological activity of hepatocyte growth factor.

### Solution to Problem

The present inventors have found that trisulfide compounds enhance the binding between HGF and its receptor c-Met, and as a result, the cell proliferation action of HGF is enhanced in a concentration-dependent manner, thereby completing the present invention.

The present invention relates, for example, to the following [1] to [23].
[1] An enhancer of physiological activity of hepatocyte growth factor, comprising a trisulfide compound,
   wherein the trisulfide compound is at least one selected from the group consisting of:
   glutathione trisulfide and a pharmaceutically acceptable salt thereof;
   a compound represented by Formula (1): 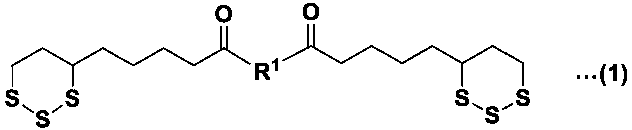
   wherein R¹ is a group represented by Formula (2): 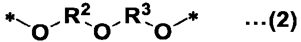
   wherein R² and R³ are each independently a C₁₋₄ alkylene group, and * is a bond;
   a group represented by Formula (3): 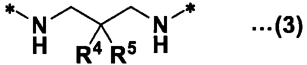
   wherein R⁴ and R⁵ are each independently a hydrogen atom or a C₁₋₄ alkyl group optionally having a substituent, R⁴ and R⁵ may form a ring together with the atom bonded thereto, and * is a bond;
   a group represented by Formula (4): 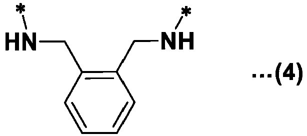
   wherein * is a bond; or
   a group represented by Formula (5): 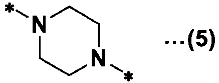
   wherein * is a bond, a pharmaceutically acceptable salt thereof, and a cyclodextrin clathrate thereof; and
   a compound represented by Formula (6): 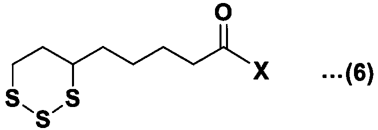
   wherein X is -OR⁶ or -NR⁷R⁸,
   wherein R⁶, R⁷, and R⁸ are each independently a hydrogen atom; a C₁₋₆ alkyl group optionally having at least one substituent selected from the group consisting of a carboxy group, -OR⁹, -NR¹⁰R¹¹, and - N⁺R¹²R¹³R¹⁴; or -(CH₂CH₂O)ₙR¹⁵,
   wherein R⁹, R¹⁰, R¹¹, R¹², R¹³, R¹⁴, and R¹⁵ are each independently a hydrogen atom or a C₁₋₃ alkyl group, and n is an integer of 2 to 5, a pharmaceutically acceptable salt thereof, and a cyclodextrin clathrate thereof.
[2] The enhancer of physiological activity according to [1], further comprising hepatocyte growth factor.
[3] An enhancer of physiological activity of hepatocyte growth factor, which is administered in combination with hepatocyte growth factor, comprising a trisulfide compound, wherein the trisulfide compound is at least one selected from the group consisting of: glutathione trisulfide and a pharmaceutically acceptable salt thereof; compound (1), a pharmaceutically acceptable salt thereof, and a cyclodextrin clathrate thereof; and compound (6), a pharmaceutically acceptable salt thereof, and a cyclodextrin clathrate thereof.
[4] An enhancer of physiological activity of hepatocyte growth factor, which is administered in combination with a trisulfide compound, comprising hepatocyte growth factor, wherein the trisulfide compound is at least one selected from the group consisting of: glutathione trisulfide and a pharmaceutically acceptable salt thereof; compound (1), a pharmaceutically acceptable salt thereof, and a cyclodextrin clathrate thereof; and compound (6), a pharmaceutically acceptable salt thereof, and a cyclodextrin clathrate thereof.
[5] The enhancer of physiological activity according to [3] or [4], wherein the trisulfide compound and hepatocyte growth factor are administered simultaneously or separately.
[6] The enhancer of physiological activity according to any one of [1] to [5], which is an enhancer for the binding between hepatocyte growth factor and c-Met.
[7] A kit for enhancing the physiological activity of hepatocyte growth factor, comprising a preparation comprising a trisulfide compound and a preparation comprising hepatocyte growth factor, wherein the trisulfide compound is at least one selected from the group consisting of: glutathione trisulfide and a pharmaceutically acceptable salt thereof; compound (1), a pharmaceutically acceptable salt thereof, and a cyclodextrin clathrate thereof; and compound (6), a pharmaceutically acceptable salt thereof, and a cyclodextrin clathrate thereof.
[8] The kit according to [7], wherein the preparation comprising the trisulfide compound and the preparation comprising hepatocyte growth factor are administered simultaneously or separately.
[9] The kit according to [7] or [8], which is for enhancing the binding between hepatocyte growth factor and c-Met.
[10] The enhancer of physiological activity or kit according to any one of [1] to [9], wherein the trisulfide compound is lipoic acid trisulfide or a pharmaceutically acceptable salt thereof.
[11] The enhancer of physiological activity or kit according to any one of [1] to [9], wherein the trisulfide compound is glutathione trisulfide or a pharmaceutically acceptable salt thereof.
[12] A method for enhancing the physiological activity of hepatocyte growth factor, comprising administering a trisulfide compound to a subject in need thereof, wherein the trisulfide compound is at least one selected from the group consisting of: glutathione trisulfide and a pharmaceutically acceptable salt thereof; compound (1), a pharmaceutically acceptable salt thereof, and a cyclodextrin clathrate thereof; and compound (6), a pharmaceutically acceptable salt thereof, and a cyclodextrin clathrate thereof.
[13] A trisulfide compound for use in enhancing the physiological activity of hepatocyte growth factor, wherein the trisulfide compound is at least one selected from the group consisting of: glutathione trisulfide and a pharmaceutically acceptable salt thereof; compound (1), a pharmaceutically acceptable salt thereof, and a cyclodextrin clathrate thereof; and compound (6), a pharmaceutically acceptable salt thereof, and a cyclodextrin clathrate thereof.
[14] Use of a trisulfide compound for the manufacture of an enhancer of physiological activity of hepatocyte growth factor, wherein the trisulfide compound is at least one selected from the group consisting of: glutathione trisulfide and a pharmaceutically acceptable salt thereof; compound (1), a pharmaceutically acceptable salt thereof, and a cyclodextrin clathrate thereof; and compound (6), a pharmaceutically acceptable salt thereof, and a cyclodextrin clathrate thereof.
[15] The method, the trisulfide compound for use, or the use according to any one of [12] to [14], wherein the enhancement of the physiological activity of hepatocyte growth factor is enhancement of the binding between hepatocyte growth factor and c-Met.
[16] A method for enhancing the physiological activity of hepatocyte growth factor, comprising administering a trisulfide compound and hepatocyte growth factor to a patient in need thereof, wherein the trisulfide compound is at least one selected from the group consisting of: glutathione trisulfide and a pharmaceutically acceptable salt thereof; compound (1), a pharmaceutically acceptable salt thereof, and a cyclodextrin clathrate thereof; and compound (6), a pharmaceutically acceptable salt thereof, and a cyclodextrin clathrate thereof.
[17] Hepatocyte growth factor for use in a method for enhancing the physiological activity of hepatocyte growth factor, which is administered in combination with a trisulfide compound, wherein the trisulfide compound is at least one selected from the group consisting of: glutathione trisulfide and a pharmaceutically acceptable salt thereof; compound (1), a pharmaceutically acceptable salt thereof, and a cyclodextrin clathrate thereof; and compound (6), a pharmaceutically acceptable salt thereof, and a cyclodextrin clathrate thereof.
[18] A trisulfide compound for use in enhancing the physiological activity of hepatocyte growth factor, which is administered in combination with hepatocyte growth factor, wherein the trisulfide compound is at least one selected from the group consisting of: glutathione trisulfide and a pharmaceutically acceptable salt thereof; compound (1), a pharmaceutically acceptable salt thereof, and a cyclodextrin clathrate thereof; and compound (6), a pharmaceutically acceptable salt thereof, and a cyclodextrin clathrate thereof.
[19] A combination of a trisulfide compound and hepatocyte growth factor for use in enhancing the physiological activity of hepatocyte growth factor, wherein the trisulfide compound is at least one selected from the group consisting of: glutathione trisulfide and a pharmaceutically acceptable salt thereof; compound (1), a pharmaceutically acceptable salt thereof, and a cyclodextrin clathrate thereof; and compound (6), a pharmaceutically acceptable salt thereof, and a cyclodextrin clathrate thereof.
[20] Use of hepatocyte growth factor for the manufacture of an enhancer of physiological activity of hepatocyte growth factor, which is administered in combination with a trisulfide compound, wherein the trisulfide compound is at least one selected from the group consisting of: glutathione trisulfide and a pharmaceutically acceptable salt thereof; compound (1), a pharmaceutically acceptable salt thereof, and a cyclodextrin clathrate thereof; and compound (6), a pharmaceutically acceptable salt thereof, and a cyclodextrin clathrate thereof.
[21] Use of a trisulfide compound for the manufacture of an enhancer of physiological activity of hepatocyte growth factor, which is administered in combination with hepatocyte growth factor, wherein the trisulfide compound is at least one selected from the group consisting of: glutathione trisulfide and a pharmaceutically acceptable salt thereof; compound (1), a pharmaceutically acceptable salt thereof, and a cyclodextrin clathrate thereof; and compound (6), a pharmaceutically acceptable salt thereof, and a cyclodextrin clathrate thereof.
[22] The method, hepatocyte growth factor for use, the trisulfide compound for use, the combination, or the use according to any one of [16] to [21], wherein the trisulfide compound and hepatocyte growth factor are administered simultaneously or separately.
[23] Use of a combination of hepatocyte growth factor and a trisulfide compound for the manufacture of an enhancer of physiological activity of hepatocyte growth factor, wherein the trisulfide compound is at least one selected from the group consisting of: glutathione trisulfide and a pharmaceutically acceptable salt thereof; compound (1), a pharmaceutically acceptable salt thereof, and a cyclodextrin clathrate thereof; and compound (6), a pharmaceutically acceptable salt thereof, and a cyclodextrin clathrate thereof.

### Advantageous Effects of Invention

According to the present invention, it is possible to provide an enhancer of physiological activity of hepatocyte growth factor.

### Brief Description of Drawings

FIG. 1 is a graph showing the effect of the addition of GSSSG or LA-SSS on the binding (affinity) between HGF and c-Met.
FIG. 2 is a graph showing the effect of the addition of GSSSG or LA-SSS on the activation effect of HGF (3 ng/mL) on muscle stem cells.
FIG. 3 is a representative bright-field microscopic image of cultured cells in Example 2.
FIG. 4 is a graph showing the effect of the addition of GSSSG or LA-SSS on the activation effect of HGF (1 ng/mL) on muscle stem cells.

### Description of Embodiments

The present invention will be described in detail below, but the present invention is not limited to the following embodiments.

The enhancer of physiological activity of hepatocyte growth factor and the method for enhancing the physiological activity of hepatocyte growth factor according to the present invention can be administered or applied to humans, pet animals, livestock, or poultry.

The trisulfide compound contained in the enhancer of physiological activity of hepatocyte growth factor according to the present invention is at least one selected from the group consisting of: glutathione trisulfide and a pharmaceutically acceptable salt thereof; compound (1), a pharmaceutically acceptable salt thereof, and a cyclodextrin clathrate thereof; and compound (6), a pharmaceutically acceptable salt thereof, and a cyclodextrin clathrate thereof.

Compound (1) can be described as a compound in which two molecules of lipoic acid trisulfide are linked via a linker.

In compound (1), R² and R³ are each independently a C₁₋₄ alkylene group. Examples of C₁₋₄ alkylene groups include methylene, ethylene, n-propylene, isopropylene, cyclopropylene, n-butylene, isobutylene, sec-butylene, tert-butylene, and cyclobutylene.

R⁴ and R⁵ are each independently a hydrogen atom or a C₁₋₄ alkyl group optionally having a substituent, and R⁴ and R⁵ may form a ring together with the atom to which they are bonded. Examples of substituents include a hydroxy group; C₁₋₄ alkoxy groups such as methoxy, ethoxy, propoxy, and butoxy. Examples of C₁₋₄ alkyl groups include methyl, ethyl, n-propyl, isopropyl, cyclopropyl, n-butyl, isobutyl, sec-butyl, tert-butyl, cyclobutyl and the like. When R⁴ and R⁵ form a ring together with the atom to which they are bonded, the formed ring may be, for example, an oxetane ring.

Compound (1) can be produced by:
(A) a step of condensing a compound represented by Formula (1a) (lipoic acid trisulfide) with a molecule for forming a linker (a compound represented by Formula (2a), a compound represented by Formula (3a), o-xylene diamine, m-xylene diamine, p-xylene diamine, piperazine, etc.) (Step 1), or
(B) a step of condensing a compound represented by Formula (1b) (lipoic acid) with a molecule for forming a linker as described above (Step 1'), a step of oxidizing the obtained disulfide compound with an oxidizing agent to obtain a sulfoxide compound (Step 2), and a step of allowing the obtained sulfoxide compound to react with a sulfur source to obtain a trisulfide compound (Step 3).

Examples of solvents used in Step 1 and Step 1' include methylene chloride, chloroform, and tetrahydrofuran, and methylene chloride is preferred. The amount of solvent may be 1 mL to 200 mL, preferably 3 mL to 35 mL, per 1 g of compound (1a) or compound (1b). Examples of condensing agents used in Step 1 and Step 1' include 1-(3-dimethylaminopropyl)-3-ethylcarbodiimide (EDC) and a salt thereof, N,N'-dicyclohexylcarbodiimide (DCC), diisopropylcarbodiimide (DIC), 4-dimethylaminopyridine (DMAP), 1,1'-carbonyl diimidazole di(1H-imidazole-1-yl)methanone (CDI) and the like. When using these compounds as condensing agents, additives such as N-hydroxysuccinimide (NHS) and 1-hydroxybenzotriazole (HOBt) may be used. The amount of condensing agent used in Step 1 and Step 1' may be 0.8 equivalents to 2.0 equivalents, preferably 1.0 equivalent to 1.5 equivalents, per 1 equivalent of compound (1a) or compound (1b). The reaction temperature in Step 1 and Step 1' may be -10°C to 40°C, preferably 15°C to 25°C. The reaction time in Step 1 and Step 1' may be 1 hour to 3 days, preferably 1 hour to 24 hours.

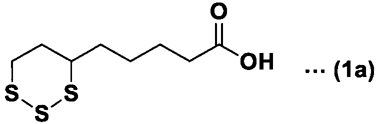

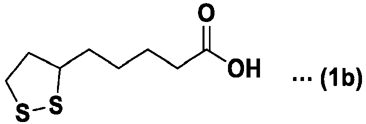

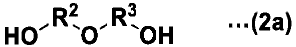

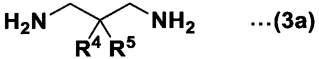

To link two molecules of compound (1a) or compound (1b) via a linker, the amount of the molecule for forming the linker used in Step 1 and Step 1' may be 0.45 to 1.00 equivalents relative to the amount of lipoic acid trisulfide. However, other equivalents may be used as long as the desired compound can be obtained.

The compound represented by Formula (1a) (lipoic acid trisulfide) can be produced by the method described in Patent Literature 2. The procedures and conditions for Steps 2 and 3 in production method (B) can be, for example, in accordance with the procedures and conditions for Steps 1 and 2 in Patent Literature 2.

R² and R³ in Formula (2a) are the same as R² and R³ in Formula (2), respectively. Examples of compounds represented by Formula (2a) include C₄₋₆ dialkylene glycols. Examples of compounds represented by Formula (2a) include diethylene glycol, linear or branched dipropylene glycol, and the like.

R⁴ and R⁵ in Formula (3a) are the same as R⁴ and R⁵ in Formula (3), respectively. Examples of compounds represented by Formula (3a) include 2,2-dimethyl-1,3-propanediamine, 2-oxetane-1,3-propanediamine, 2-methoxy-1,3-propanediamine, and the like.

Examples of compound (1) include compounds represented by the following Formulae (11) to (16) (compounds (11) to (16)) or the like.

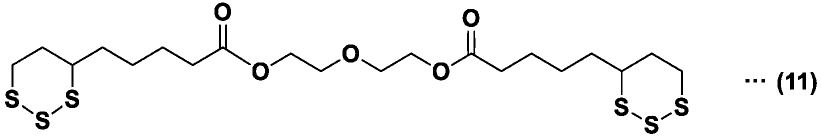

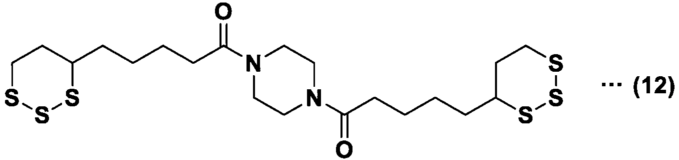

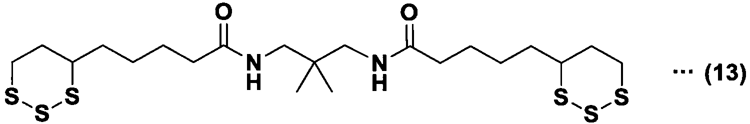

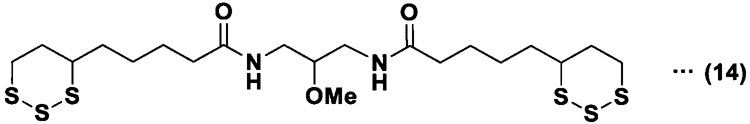

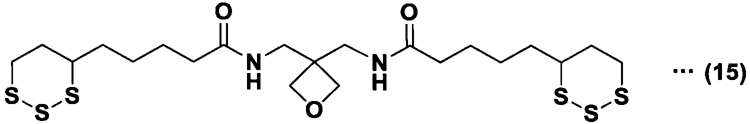

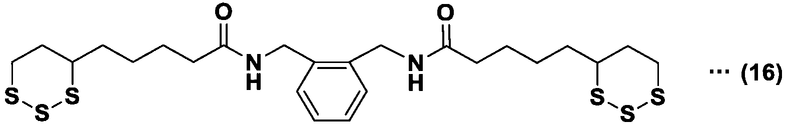

The molecular weights and ClogP values of compounds (11) to (16) are shown in Table 1.

**[Table 1]**

| | Compound (11) | Compound (12) | Compound (13) | Compound (14) | Compound (15) | Compound (16) |
|---|---|---|---|---|---|---|
| M.W. | 546.8 | 526.9 | 542.9 | 544.9 | 556.9 | 576.9 |
| ClogP | 6.512 | 4.792 | 5.585 | 4.568 | 3.381 | 5.732 |

The molecular weight of compound (1) or a salt thereof may be 800 or less, 750 or less, or 700 or less, and is preferably 600 or less. The molecular weight of compound (1) or a salt thereof may be 400 or more, 450 or more, or 500 or more. When the molecular weight of compound (1) or a salt thereof is within these ranges, the *in vivo* absorption rate is considered to be further improved.

The ClogP of compound (1) or a salt thereof may be 1 or more, 2 or more, 3 or more, or 4 or more. The ClogP of compound (1) or a salt thereof may be 8 or less, 7 or less, or 6 or less. When the ClogP of compound (1) or a salt thereof is within these ranges, the balance of water solubility and lipophilicity of compound (1) or a salt thereof is considered to be more excellent. ClogP is a computer-calculated partition coefficient and can be determined in accordance with the principles described in "CLOGP Reference Manual Daylight Version 4.9 (Release date: August 1, 2011, https://www.daylight.com/dayhtml/doc/clogp/)".

A salt of compound (1) may be a pharmacologically acceptable salt, and examples thereof include salts with alkali metals such as sodium and potassium; salts with alkaline earth metals such as calcium and magnesium; ammonium salts; salts with inorganic acids such as hydrochloric acid, hydrobromic acid, sulfuric acid, nitric acid, and phosphoric acid; and salts with organic acids such as acetic acid, succinic acid, fumaric acid, maleic acid, tartaric acid, citric acid, lactic acid, stearic acid, benzoic acid, methanesulfonic acid, ethanesulfonic acid, and p-toluenesulfonic acid. When compound (1) is obtained as a free form, it can be converted to a salt by a conventional method. When compound (1) is obtained as a salt, it can also be converted to a free form by a conventional method.

The compounds described in this specification may have optical isomers (R-form, S-form, R,R-form, R,S-form, S,R-form, and S,S-form). The compounds described herein may be replaced with their optical isomers or mixtures containing the optical isomers at any ratio (for example, racemates). For example, compound (1) may be an optical isomer represented by Formula (1-1), an optical isomer represented by Formula (1-2), an optical isomer represented by Formula (1-3), or an optical isomer represented by Formula (1-4), or may be replaced with a mixture containing these optical isomers at any ratio (for example, racemate).

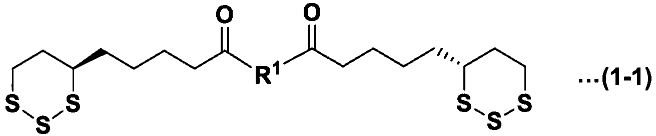

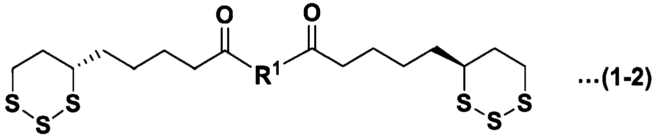

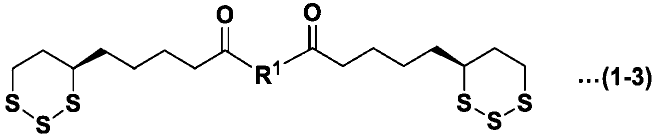

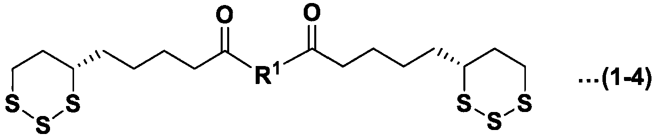

Compound (6) may be, in one embodiment, a compound represented by Formula (7): 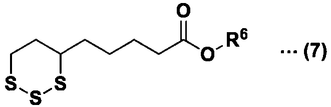 wherein R⁶ is a hydrogen atom; a C₁₋₆ alkyl group optionally having at least one substituent selected from the group consisting of a carboxy group, -OR⁹, -NR¹⁰R¹¹, and -N⁺R¹²R¹³R¹⁴; or -(CH₂CH₂O)ₙR¹⁵, wherein R⁹, R¹⁰, R¹¹, R¹², R¹³, R¹⁴, and R¹⁵ are each independently a hydrogen atom or a C₁₋₃ alkyl group, and n is an integer of 2 to 5. For example, R⁶ may be a hydrogen atom, methyl, ethyl, propyl, butyl, pentyl, or hexyl. For example, R⁶ is a hydrogen atom, in which case the compound represented by Formula (7) is lipoic acid trisulfide represented by Formula (8).

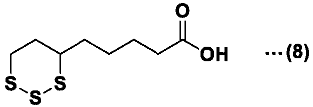

Compound (6) may be, in another embodiment, a compound represented by Formula (9): 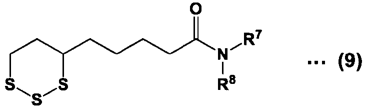 wherein R⁷ and R⁸ are each independently a hydrogen atom; a C₁₋₆ alkyl group optionally having at least one substituent selected from the group consisting of a carboxy group, -OR⁹, -NR¹⁰R¹¹, and -N⁺R¹²R¹³R¹⁴; or -(CH₂CH₂O)ₙR¹⁵, wherein R⁹, R¹⁰, R¹¹, R¹², R¹³, R¹⁴, and R¹⁵ are each independently a hydrogen atom or a C₁₋₃ alkyl group, and n is an integer of 2 to 5. R⁷ and R⁸ may be a hydrogen atom or an alkyl group such as methyl, ethyl, propyl, butyl, pentyl, or hexyl. These alkyl groups may have one or both of an amino group and a carboxy group as substituents. R⁷ and R⁸ may be, for example, a group represented by Formula (10), wherein * is a bond. Specific examples of the compound represented by Formula (9) include a compound in which both R⁷ and R⁸ are hydrogen atoms, and a compound in which R⁷ is a hydrogen atom and R⁸ is a group represented by Formula (10).

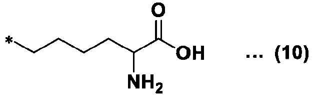

The compound represented by Formula (9) can be produced by a step (Step 61) of oxidizing a compound represented by Formula (9a) with an oxidizing agent to obtain a sulfoxide compound, and a step (Step 62) of reacting the obtained sulfoxide compound with a sulfur source. 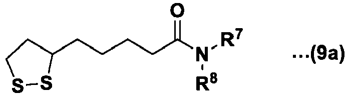 wherein R⁷ and R⁸ are each independently a hydrogen atom; a C₁₋₆ alkyl group optionally having at least one substituent selected from the group consisting of a carboxy group, -OR⁹, -NR¹⁰R¹¹, and -N⁺R¹²R¹³R¹⁴; or -(CH₂CH₂O)ₙR¹⁵, wherein R⁹, R¹⁰, R¹¹, R¹², R¹³, R¹⁴, and R¹⁵ are each independently a hydrogen atom or a C₁₋₃ alkyl group, and n is an integer of 2 to 5.

In the above production method, Steps 61 and 62 may be carried out in one pot without isolating the sulfoxide compound.

The solvent used in Step 61 is not particularly limited as long as it dissolves the compound represented by Formula (9a) and the oxidizing agent and does not inhibit the oxidation reaction. Examples of such solvents include water, aqueous sulfuric acid solution, aqueous ethanol solution, and aqueous acetonitrile solution, and water is preferred. The amount of solvent used in Step 61 may be 1 mL to 500 mL, preferably 10 mL to 20 mL, per 1 g of the compound represented by Formula (9a).

Examples of oxidizing agents used in Step 61 include potassium peroxymonosulfate (sold under trade names such as Oxone^{®}), peracetic acid, hydrogen peroxide, and sodium periodate. Hydrogen peroxide may be used with a catalytic amount of methyltrioxorhenium. From the viewpoint of safety and cost, potassium peroxymonosulfate is a preferred oxidizing agent. The amount of oxidizing agent used may be 0.8 equivalents to 2.0 equivalents, preferably 1.0 equivalent to 1.3 equivalents, per 1 equivalent of the compound represented by Formula (9a).

The reaction temperature of Step 61 may be -20°C to 30°C, preferably -5°C to 5°C.

The reaction time of Step 61 may be 5 minutes to 24 hours, preferably 0.5 hours to 2 hours.

The solvent used in Step 62 is not particularly limited as long as it dissolves the sulfoxide compound and the sulfur source and does not inhibit the subsequent reaction. Examples of such solvents include water, aqueous sulfuric acid solution, aqueous ethanol solution, and aqueous acetonitrile solution, and water is preferred. The amount of solvent used in Step 62 may be 1 mL to 500 mL, preferably 10 mL to 20 mL, per 1 g of the sulfoxide compound.

Examples of sulfur sources used in Step 62 include sodium sulfide, potassium sulfide, sodium hydrogen sulfide, potassium hydrogen sulfide, and hydrogen sulfide. The amount of sulfur source used may be 0.5 equivalents to 4.0 equivalents, preferably 0.9 equivalents to 1.2 equivalents, per 1 equivalent of the sulfoxide compound.

The reaction temperature of Step 62 may be -20°C to 30°C, preferably -5°C to 25°C.

The reaction time of Step 62 may be 10 minutes to 2 days, preferably 0.5 hours to 2 hours.

When Steps 61 and 62 are carried out in one pot, examples of reaction solvents include water, aqueous sulfuric acid solution, aqueous ethanol solution, and aqueous acetonitrile solution, and water is preferred. The amount of solvent may be 1 mL to 500 mL, preferably 10 mL to 20 mL, per 1 g of the compound represented by Formula (9a). Examples of oxidizing agents used include potassium peroxymonosulfate, peracetic acid, hydrogen peroxide (which may be used with a catalytic amount of methyltrioxorhenium), and sodium periodate, and potassium peroxymonosulfate is preferred. The amount of oxidizing agent used may be 0.8 equivalents to 2.0 equivalents, preferably 1.0 equivalent to 1.3 equivalents, per 1 equivalent of the compound represented by Formula (9a). Examples of sulfur sources used include sodium sulfide, potassium sulfide, sodium hydrogen sulfide, potassium hydrogen sulfide, and hydrogen sulfide. The amount of sulfur source used may be 0.5 equivalents to 4.0 equivalents, preferably 0.9 equivalents to 1.2 equivalents, per 1 equivalent of the compound represented by Formula (9a). The reaction temperature may be -20°C to 30°C, preferably -5°C to 25°C. The reaction time may be 15 minutes to 2 days, preferably 1 hour to 4 hours.

In addition to Steps 61 and 62, the method may include, if necessary, a step of protecting functional groups such as a hydroxyl group, a carbonyl group, an amino group, and a carboxyl group, and a step of deprotecting the protected functional groups. Protective groups for these functional groups and protection/deprotection reactions are well known to those skilled in the art, and appropriate protective groups and protection/deprotection reactions can be selected with reference to "Greene's Protective Groups in Organic Synthesis" or the like.

The compound represented by Formula (9a) can be produced by condensing lipoic acid with NHR⁷R⁸. Examples of solvents for the condensation reaction include dichloromethane, chloroform, and tetrahydrofuran, and tetrahydrofuran is preferred. The amount of solvent may be 1 mL to 200 mL, preferably 3 mL to 35 mL, per 1 g of the compound represented by Formula (9a). Examples of condensing agents used include 1-(3-dimethylaminopropyl)-3-ethylcarbodiimide (EDC) and a salt thereof, N,N'-dicyclohexylcarbodiimide (DCC), and diisopropylcarbodiimide (DIC) (N-hydroxysuccinimide (NHS) and 1-hydroxybenzotriazole (HOBt) may be used as additives). The amount of condensing agent used may be 0.8 equivalents to 2.0 equivalents, preferably 1.0 equivalent to 1.5 equivalents, per 1 equivalent of the compound represented by Formula (9a). The reaction temperature may be -10°C to 40°C, preferably 15°C to 25°C. The reaction time may be 1 hour to 3 days, preferably 1 hour to 24 hours.

The compound represented by Formula (9) can also be produced by condensing lipoic acid trisulfide with NHR⁷R⁸. The conditions for condensation are the same as described above.

The compound represented by Formula (7) can be produced by a step (Step 61) of oxidizing a compound represented by Formula (7a) with an oxidizing agent to obtain a sulfoxide compound, and a step (Step 62) of reacting the obtained sulfoxide compound with a sulfur source. The reaction conditions are the same as described above. 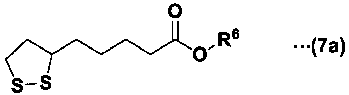 wherein R⁶ is a hydrogen atom; a C₁₋₆ alkyl group optionally having at least one substituent selected from the group consisting of a carboxy group, -OR⁹, -NR¹⁰R¹¹, and -N⁺R¹²R¹³R¹⁴; or -(CH₂CH₂O)ₙR¹⁵, wherein R⁹, R¹⁰, R¹¹, R¹², R¹³, R¹⁴, and R¹⁵ are each independently a hydrogen atom or a C₁₋₃ alkyl group, and n is an integer of 2 to 5.

The compound represented by Formula (7a) can be produced by condensing lipoic acid with R⁶OH. The conditions are the same as described above.

The compound represented by Formula (7) can also be produced by condensing lipoic acid trisulfide with R⁶OH. The conditions for condensation are the same as described above.

In the present invention, examples of pharmaceutically acceptable salts include salts with inorganic acids such as hydrochloric acid, hydrobromic acid, sulfuric acid, nitric acid, and phosphoric acid; salts with organic acids such as acetic acid, succinic acid, fumaric acid, maleic acid, tartaric acid, citric acid, lactic acid, stearic acid, benzoic acid, methanesulfonic acid, ethanesulfonic acid, and p-toluenesulfonic acid; salts with alkali metals such as sodium and potassium; salts with alkaline earth metals such as calcium and magnesium; ammonium salts; and salts with amino acids such as arginine.

The pharmaceutically acceptable salt of glutathione trisulfide is preferably an amino acid salt or an alkali metal salt, more preferably an arginine salt or a sodium salt. The pharmaceutically acceptable salt of compound (1) and the pharmaceutically acceptable salt of compound (6) are preferably salts with alkali metals, more preferably sodium salts.

Glutathione trisulfide or a pharmaceutically acceptable salt thereof, compound (1) or a pharmaceutically acceptable salt thereof, or compound (6) or a pharmaceutically acceptable salt thereof may have crystal polymorphs, but is not limited to any particular crystal form, and may be a single crystal form or a mixture of crystal forms. Glutathione trisulfide or a pharmaceutically acceptable salt thereof, compound (1) or a pharmaceutically acceptable salt thereof, or compound (6) or a pharmaceutically acceptable salt thereof also includes amorphous forms. Glutathione trisulfide or a pharmaceutically acceptable salt thereof, compound (1) or a pharmaceutically acceptable salt thereof, or compound (6) or a pharmaceutically acceptable salt thereof includes anhydrates and solvates (especially hydrates).

The trisulfide compound may be a cyclodextrin clathrate (CD clathrate) in which at least one selected from the group consisting of compound (1) and a pharmaceutically acceptable salt thereof, and compound (6) and a pharmaceutically acceptable salt thereof is included in a cyclodextrin.

The cyclodextrin may be α-cyclodextrin, β-cyclodextrin, γ-cyclodextrin, or a derivative thereof. The term "derivative" means that a hydrogen atom of at least one hydroxyl group of each cyclodextrin is substituted with an alkyl group optionally having a substituent or a saccharide. Examples of cyclodextrin derivatives include methyl-α-cyclodextrin, methyl-β-cyclodextrin, methyl-γ-cyclodextrin, dimethyl-α-cyclodextrin, dimethyl-β-cyclodextrin, dimethyl-γ-cyclodextrin, hydroxyethyl-α-cyclodextrin, hydroxyethyl-β-cyclodextrin, hydroxyethyl-γ-cyclodextrin, 2-hydroxypropyl-α-cyclodextrin, 2-hydroxypropyl-β-cyclodextrin, 2-hydroxypropyl-γ-cyclodextrin, glucosyl-α-cyclodextrin, glucosyl-β-cyclodextrin, glucosyl-γ-cyclodextrin, maltosyl-α-cyclodextrin, maltosyl-β-cyclodextrin, maltosyl-γ-cyclodextrin, sulfobutyl ether-β-cyclodextrin and the like.

The cyclodextrin clathrate can be produced by a step (Step a) of dissolving cyclodextrin in a solvent, a step (Step b) of adding at least one selected from the group consisting of compound (1) and a pharmaceutically acceptable salt thereof, and compound (6) and a pharmaceutically acceptable salt thereof to the obtained solution and stirring, and a step (Step c) of filtering the stirred solution, washing with the same solvent as used in Step a, freezing the filtrate, and freeze-drying. The filtration and washing operation in Step c may be omitted.

The solvent used in Step a is preferably water.

The amount of solvent used in Step a may be 1 to 350 mL, preferably 1 to 80 mL, per 1 g of cyclodextrin.

In Step b, the mass ratio of cyclodextrin to at least one selected from the group consisting of compound (1) and a pharmaceutically acceptable salt thereof, and compound (6) and a pharmaceutically acceptable salt thereof may be 2 to 20, preferably 5 to 16.5.

The stirring temperature in Step b may be 20 to 50°C, and may be room temperature.

The stirring time in Step b may be 0.25 to 40 hours, preferably 2 to 35 hours.

In Step b, after adding at least one selected from the group consisting of compound (1) and a pharmaceutically acceptable salt thereof, and compound (6) and a pharmaceutically acceptable salt thereof, the same solvent as used in Step a may be added before stirring. In this case, the amount of solvent may be 0 to 30 mL, preferably 0 to 20 mL, per 1 g of cyclodextrin.

The amount of solvent used in Step c may be 0 to 150 mL, preferably 0 to 20 mL, per 1 g of cyclodextrin.

The freezing temperature in Step c may be -30 to -20°C, preferably -20°C.

The freezing time in Step c may be 10 to 50 hours.

The freeze-drying in Step c may be carried out at an absolute pressure of 20 to 100 Pa, with an external temperature of 10 to 40°C, preferably an external temperature of 20°C.

The freeze-drying period in Step c may be 1 to 5 days.

The enhancer of physiological activity of hepatocyte growth factor is considered to be able to enhance the cell proliferation-promoting action of hepatocyte growth factor on hepatocytes, renal tubular epithelial cells, mammary epithelial cells, bronchial epithelial cells, bile duct epithelial cells, keratinocytes, melanocytes, vascular endothelial cells, dermal fibroblasts, chondrocytes, osteoclasts, muscle satellite cells, mesenchymal stem cells, and the like. The enhancer of physiological activity of hepatocyte growth factor is also considered to exhibit neuroprotective action. The enhancement of the physiological activity of hepatocyte growth factor may be to increase the affinity of hepatocyte growth factor for c-Met. c-Met is a receptor for hepatocyte growth factor, and the cell proliferation-promoting action on the above-mentioned cells is considered to be exerted through the binding of HGF and c-Met.

In the present invention, "administered in combination" or "combined use" means using active ingredients in combination, and includes (1) administration as a single preparation containing HGF and a trisulfide compound, and (2) administration where HGF and a trisulfide compound are administered as separate preparations simultaneously or separately with a time interval. In case (2), HGF may be administered first, or the trisulfide compound may be administered first. Also, in case (2), the administration may be in any of the following forms: (i) HGF and the trisulfide compound are separately formulated and administered simultaneously via the same administration route, (ii) HGF and the trisulfide compound are separately formulated and administered separately with a time interval via the same administration route, (iii) HGF and the trisulfide compound are separately formulated and administered simultaneously via different administration routes (administered from different sites of the same patient), or (iv) HGF and the trisulfide compound are separately formulated and administered separately with a time interval via different administration routes. In case (i), both agents may be mixed immediately before administration.

In other words, "administered in combination" or "combined use" in the present invention can also be described as a mode of use in which one agent is administered while the action/effect of the other agent is manifested in the patient's body. That is, in the present invention, it is preferable that HGF and the trisulfide compound are administered so as to be present simultaneously in the patient's body, for example, in the blood, and it is preferable that one agent is administered within 24 hours after the other agent is administered to the patient.

When administered in combination with a trisulfide compound, the daily dose of HGF is preferably 0.005 to 100 mg, more preferably 0.05 to 50 mg, and even more preferably 0.1 to 10 mg, for example, for an adult.

When administered in combination with HGF, the daily dose of the trisulfide compound is preferably 0.1 to 5000 mg, more preferably 0.5 to 1000 mg, and even more preferably 1 to 500 mg, for example, for an adult.

For example, for an adult, it is preferable that the dose of HGF is 0.005 to 100 mg/day and the dose of the trisulfide compound is 0.1 to 5000 mg/day, more preferably the dose of HGF is 0.05 to 50 mg/day and the dose of the trisulfide compound is 0.5 to 1000 mg/day, and even more preferably the dose of HGF is 0.1 to 10 mg/day and the dose of the trisulfide compound is 1 to 500 mg/day. When the doses of HGF and the trisulfide compound are within these ranges, the enhancing effect on physiological activity of hepatocyte growth factor is considered to be further improved.

The number of administrations of HGF and the trisulfide compound may be 1 to 6 times per day or 1 to 4 times per day. With such a number of administrations, the burden of medication on the subject is reduced, and medication compliance is expected to improve. As a result, the enhancing effect on physiological activity of hepatocyte growth factor according to the present invention is expected to be further improved. When discontinuing the medication, the dose of the agent may be gradually reduced. For example, one agent may be administered during a drug holiday of the other agent.

The enhancer of physiological activity of hepatocyte growth factor according to the present invention can be used for the prevention or treatment of muscle atrophy and/or muscle regeneration failure.

The muscle atrophy in the present invention includes those arising from a state where muscles have not been used for a long time or a state where the frequency or intensity of use has decreased due to aging, being bedridden, trauma, postoperative fixation, zero gravity, or suffering from some disease. The muscle atrophy or muscle regeneration failure in the present invention is related to disorders in muscle growth, hypertrophy, regeneration, or maintenance, which originate from inhibition of activation or decreased proliferation of muscle stem cells (satellite cells), and can be exemplified by age-related sarcopenia, amyotrophic lateral sclerosis, spinal muscular atrophy, spinal and bulbar muscular atrophy, and muscular dystrophy as representatives.

Examples of pet animals include dogs, cats, hamsters, rabbits, ferrets, parakeets, Java sparrows, parrots, and the like.

The dose of the enhancer of physiological activity of hepatocyte growth factor, or the preventive or therapeutic agent for muscle atrophy and muscle regeneration failure according to the present invention is a therapeutically effective amount and varies depending on symptoms, age, administration route, dosage form, and the like. In ordinary cases, for adults, glutathione trisulfide or a pharmaceutically acceptable salt thereof, compound (1) or a pharmaceutically acceptable salt thereof, or compound (6) or a pharmaceutically acceptable salt thereof can be administered at 0.1 to 5000 mg per day, and it is preferable to administer 0.5 to 2000 mg per day in one or several divided doses daily. When the dose of the above compound is within this range, the enhancing effect on physiological activity of hepatocyte growth factor, and the preventive or therapeutic effect against muscle atrophy and muscle regeneration failure in humans are considered to be further improved.

The dose of the enhancer of physiological activity of hepatocyte growth factor, or the preventive or therapeutic agent for muscle atrophy and muscle regeneration failure according to the present invention is a therapeutically effective amount and varies depending on symptoms, age, administration method, dosage form, and the like. In ordinary cases, for pets, glutathione trisulfide or a pharmaceutically acceptable salt thereof, compound (1) or a pharmaceutically acceptable salt thereof, or compound (6) or a pharmaceutically acceptable salt thereof can be administered at 0.01 to 500 mg/kg per day, and it is preferable to administer 0.05 to 250 mg/kg per day in one or several divided doses daily. When the dose of the above compound is within this range, the enhancing effect on physiological activity of hepatocyte growth factor, and the preventive or therapeutic effect against muscle atrophy and muscle regeneration failure in pets are considered to be further improved.

The enhancer of physiological activity of hepatocyte growth factor according to the present invention can be used for suppressing or improving heat stress-induced inhibition of muscle growth (reduction in meat production) in livestock or poultry.

Causes of heat stress-induced inhibition of muscle growth (reduction in meat production) in livestock or poultry in the present invention include an increase in the outside air temperature of the rearing environment of livestock or poultry due to summer heat, and abnormalities or deviations in temperature control in the rearing management area of livestock or poultry. These causes are thought to induce respiratory alkalosis, decreased thyroid hormone, increased corticosterone, changes in immune response, and excessive production of mitochondrial reactive oxygen species (ROS) in the bodies of livestock or poultry. As a result, disorders in muscle growth, hypertrophy, regeneration, or maintenance are caused, starting with inhibition of activation or decreased proliferation of muscle stem cells (satellite cells), leading to a reduction in meat production.

Examples of livestock or poultry include cattle, horses, pigs, donkeys, sheep, goats, chickens, ducks, geese, ostriches, turkeys, ducks, quails, and the like.

The dose of the enhancer of physiological activity of hepatocyte growth factor, or the agent for suppressing or improving inhibition of muscle growth (reduction in meat production) in livestock or poultry according to the present invention is a therapeutically effective amount and varies depending on symptoms, administration route, and the like. In ordinary cases, for livestock or poultry, glutathione trisulfide or a pharmaceutically acceptable salt thereof, compound (1) or a pharmaceutically acceptable salt thereof, or compound (6) or a pharmaceutically acceptable salt thereof can be administered at 0.01 to 500 mg/kg per day, and it is preferable to administer 0.05 to 250 mg/kg per day in one or several divided doses daily. When the dose of the above compound is within this range, the enhancing effect on physiological activity of hepatocyte growth factor in livestock or poultry, and the effect of suppressing or improving inhibition of muscle growth (reduction in meat production) in livestock or poultry are considered to be further improved.

The enhancer of physiological activity of hepatocyte growth factor, the preventive or therapeutic agent for muscle atrophy and/or muscle regeneration failure, and the agent for suppressing or improving inhibition of muscle growth (reduction in meat production) according to the present invention can be administered orally as, for example, tablets, capsules, powders, granules, liquids, or syrups, or parenterally as injections, infusions, or suppositories. The enhancer of physiological activity of hepatocyte growth factor, the preventive or therapeutic agent for muscle atrophy and/or muscle regeneration failure, and the agent for suppressing or improving inhibition of muscle growth (reduction in meat production) according to the present invention can be formulated into these dosage forms by known formulation techniques with the addition of pharmaceutically acceptable additives as necessary. In the case of solid preparations, pharmaceutically acceptable excipients such as starch, lactose, refined sugar, glucose, crystalline cellulose, carboxylcellulose, carboxymethylcellulose, carboxyethylcellulose, calcium phosphate, magnesium stearate, and gum arabic can be incorporated during formulation, and if necessary, lubricants, binders, disintegrants, coating agents, colorants, and the like can be incorporated. In the case of liquid preparations, stabilizers, solubilizing agents, suspending agents, emulsifiers, buffers, preservatives, and the like can be incorporated.

### [Examples]

Hereinafter, the present invention will be described in detail with reference to examples, but the present invention is not limited by these examples.

### Example 1

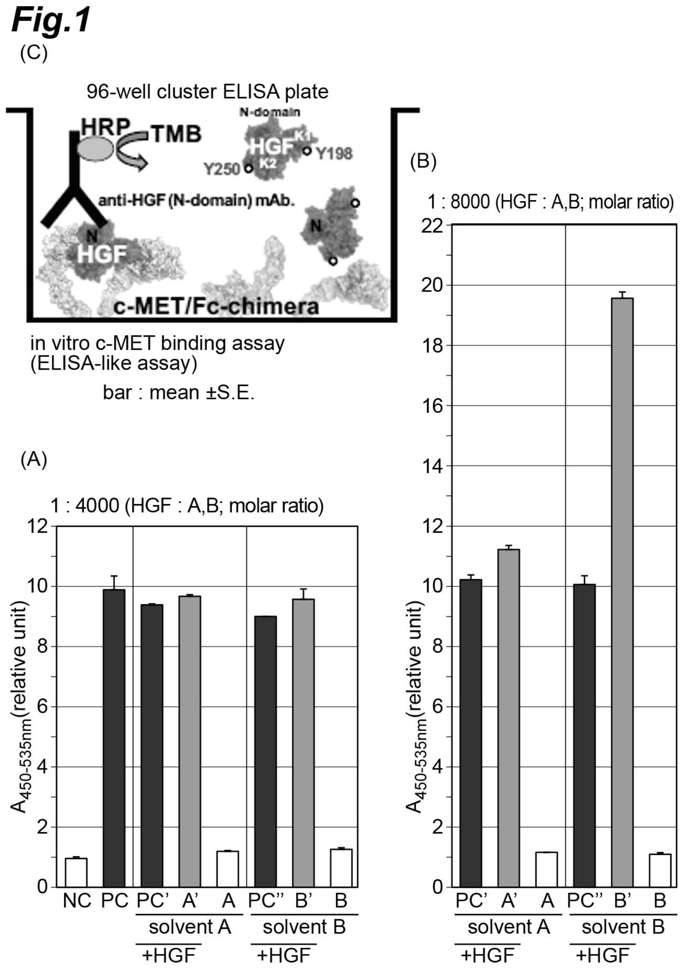

The effect of the addition of glutathione trisulfide (GSSSG) or lipoic acid trisulfide (LA-SSS) on the binding (affinity) between HGF and c-Met was examined by the following method. GSSSG or LA-SSS was added to recombinant HGF (R&D Systems) at a molar ratio of 1:4000 (HGF:GSSSG or HGF:LA-SSS) (Fig. 1(A)) or 1:8000 (HGF:GSSSG or HGF:LA-SSS) (Fig. 1(B)). An ELISA-like HGF-c-Met binding assay was performed using a 96-well cluster plate with immobilized recombinant c-Met (R&D Systems) (Tatsumi et al. 2002; the principle of this method is schematically shown in Fig. 1(C)). Table 2 and Fig. 1(A) and (B) show the relative values of each experimental group, with the A₄₅₀₋₅₃₅ₙₘ (absorbance at 450 nm minus absorbance at 535 nm) of the negative control (NC) set to 1.

**[Table 2]**

| **HGF : A, B (Molar ratio)** | **Experimental conditions** | **A₄₅₀₋₅₃₅ₙₘ (Relative units) (Average value)** | **S.E** |
|---|---|---|---|
| - | NC | 1.00 | 0.01 |
| - | PC | 9.85 | 0.48 |
| 1 : 4000 | PC' | 9.39 | 0.06 |
| | A' (A + 1 ng/mL HGF) | 9.67 | 0.13 |
| | A | 1.21 | 0.03 |
| | PC" | 9.00 | 0.02 |
| | B' (B + 1 ng/mL HGF) | 9.55 | 0.35 |
| | B | 1.28 | 0.02 |
| | PC' | 10.21 | 0.18 |
| 1 : 8000 | A' (A + 1 ng/mL HGF) | 11.22 | 0.16 |
| | A | 1.16 | 0.01 |
| | PC" | 10.04 | 0.33 |
| | B' (B + 1 ng/mL HGF) | 19.60 | 0.18 |
| | B | 1.14 | 0.02 |

In Table 2 and Fig. 1, NC: negative control (no addition), PC: positive control (HGF only addition), PC': addition of solvent used to dissolve GSSSG and HGF, A': addition of solvent used to dissolve GSSSG, GSSSG, and HGF (1 ng/ml), A: addition of solvent used to dissolve GSSSG and GSSSG, PC": addition of solvent used to dissolve LA-SSS and HGF, B': addition of solvent used to dissolve LA-SSS, LA-SSS, and HGF (1 ng/ml), B: addition of solvent used to dissolve LA-SSS and LA-SSS.

When GSSSG or LA-SSS was added at 8000 moles per 1 mole of HGF, the A₄₅₀₋₅₃₅ₙₘ value indicating the binding between HGF and c-Met (vertical axis in Fig. 1(B)) significantly increased (p < 0.01 vs. PC' or PC"). This effect was more pronounced with LA-SSS (see A', B' in Fig. 1(B)). Even when GSSSG or LA-SSS was added at 4000 moles per 1 mole of HGF, the A₄₅₀₋₅₃₅ₙₘ value indicating the binding between HGF and c-Met (vertical axis in Fig. 1(A)) increased (p < 0.01 vs. PC' or PC"). This effect was more pronounced with LA-SSS (see bar graphs A', B' in Fig. 1(A)). Therefore, it was found that when GSSSG or LA-SSS is added to HGF at a high molar ratio, the binding affinity between HGF and c-Met significantly increases.

### Example 2

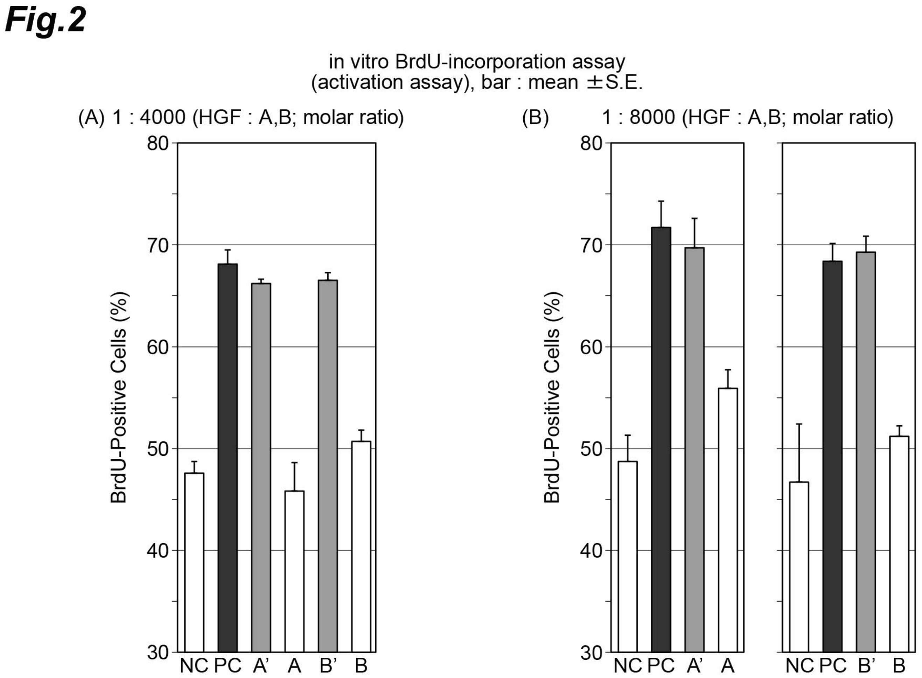

The effect of the addition of GSSSG or LA-SSS on the muscle stem cell activation effect of HGF (3 ng/ml) was examined by the following method. GSSSG or LA-SSS was added to recombinant HGF (R&D Systems) at a molar ratio of 1:4000 (HGF:GSSSG or HGF:LA-SSS) (Fig. 2(A)) or 1:8000 (HGF:GSSSG or HGF:LA-SSS) (Fig. 2(B)). The activation effect of HGF was measured using a culture system of muscle stem cells (satellite cells). Quiescent muscle stem cells were isolated from the skeletal muscle of 8-9-month-old mature rats in accordance with a modified method of Allen et al. (1997) (Tatsumi et al. 2006), and cultured for 24 hours in DMEM containing 10% normal horse serum (DMEM-10% HS, pH 7.2). HGF mixed with GSSSG or LA-SSS was added to a final concentration of 3 ng/ml, and after further culture for 24 hours, the activation rate of muscle stem cells was measured by the bromodeoxyuridine (BrdU) pulse labeling method. Cells that had incorporated BrdU were visualized with an anti-BrdU monoclonal antibody, and the percentage of positive cells was used as an indicator of muscle stem cell activation (Tatsumi et al. 1998).

In Fig. 2, NC: negative control (no addition), PC: positive control (HGF only addition), A': addition of GSSSG and HGF (3 ng/ml), A: addition of GSSSG, B': addition of LA-SSS and HGF (3 ng/ml), B: addition of LA-SSS.

When GSSSG or LA-SSS was added at 4000 moles or 8000 moles per 1 mole of HGF, the percentage of BrdU-positive cells, which is an index showing the muscle stem cell activation effect of HGF, significantly increased (p < 0.05 vs. NC), and this value was comparable to that of the positive control (PC) (see A', B' in Fig. 2(A), (B)). Therefore, it was confirmed that GSSSG and LA-SSS do not adversely affect the physiological activity of HGF and the responsiveness of muscle stem cells (such as c-Met receptor signaling activity). This was also supported by the following cell morphology observation.

When GSSSG or LA-SSS was added at 8000 moles per 1 mole of HGF, the enhancing effect seen in Example 1 was not observed. Since the amount of c-Met expression on the cell surface (number of molecules) is considered to be controlled within an appropriate range in the cell culture system, the results were considered to differ from the c-Met binding experimental system where a large amount of c-Met was immobilized on the ELISA plate.

### Example 3

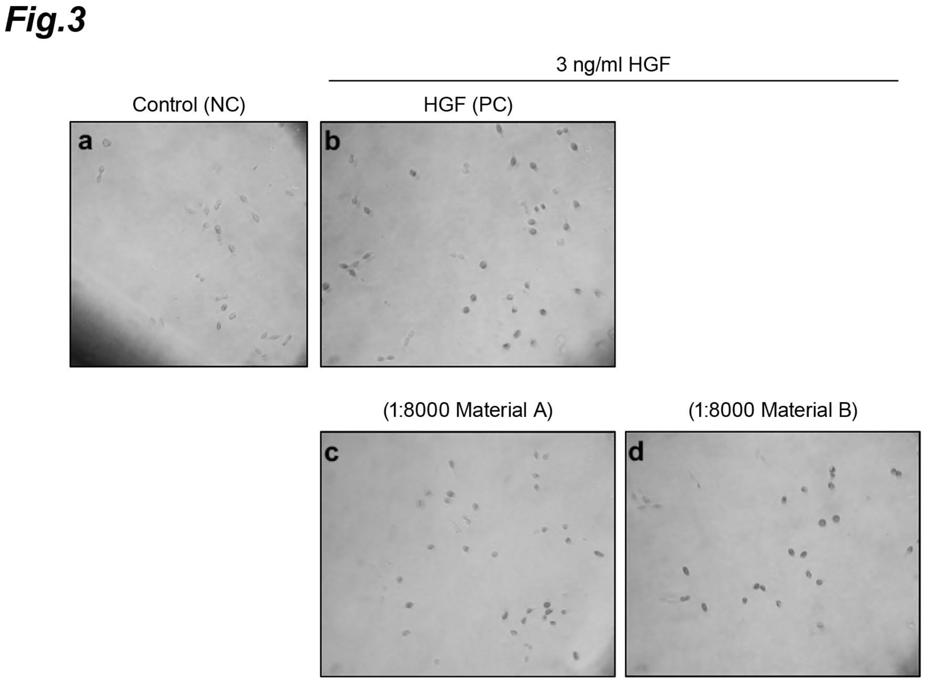

The morphology of muscle stem cells cultured with the addition of GSSSG or LA-SSS and HGF (3 ng/ml) was observed by the following method. Representative fields of the culture system used in Example 2 were photographed. Fig. 3a is a representative field of NC in Fig. 2(A), (B), Fig. 3b is a representative field of PC in Fig. 2(A), (B), Fig. 3c is a representative field of A' (A + HGF (3 ng/ml)) in Fig. 2(B), and Fig. 3d is a representative field of B' (B + HGF (3 ng/ml)) in Fig. 2(B).

As shown in Fig. 3c, d, the morphology of cells cultured with the addition of GSSSG or LA-SSS and HGF (3 ng/ml) did not differ from the morphology observed in the control groups (NC and PC: Fig. 3a, b). Therefore, it was confirmed that GSSSG and LA-SSS do not adversely affect the physiological activity of HGF and the responsiveness of muscle stem cells (such as c-Met receptor signaling activity).

### Example 4

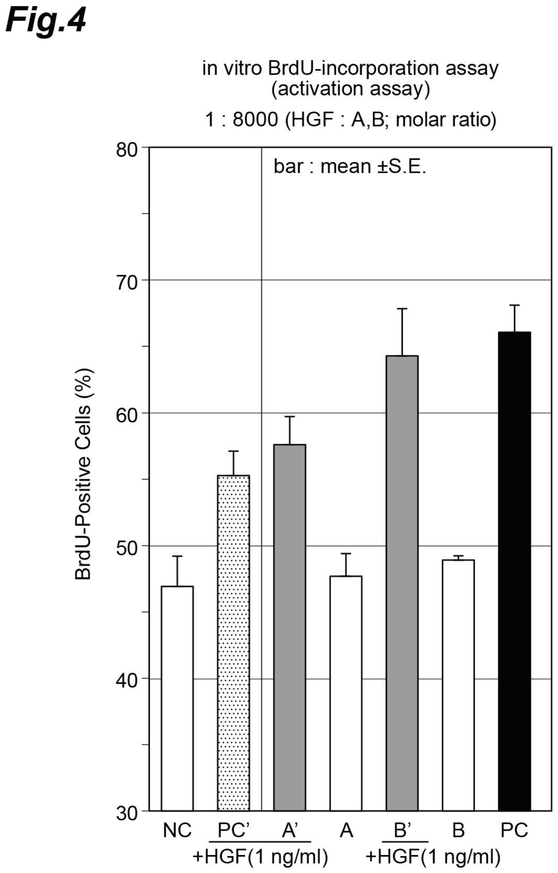

The effect of the addition of GSSSG or LA-SSS on the muscle stem cell activation effect of HGF (1 ng/ml) was examined in the same manner as in Example 2, except that the final concentration of HGF was 1 ng/ml instead of 3 ng/ml. The results are shown in Table 3 and Fig. 4.

**[Table 3]**

| **Experimental conditions** | **Percentage of BrdU-positive cells (%)** | **S.E.** |
|---|---|---|
| NC | 46.9 | 2.2 |
| PC' | 55.3 | 1.8 |
| A' (A+ 1 ng/ml HGF) | 57.5 | 2.1 |
| A | 47.5 | 1.8 |
| B' (B+ 1 ng/ml HGF) | 64.2 | 3.4 |
| B | 48.8 | 0.4 |
| PC | 65.9 | 2.2 |

In Table 3 and Fig. 4, NC: negative control (no addition), PC': positive control (HGF (1 ng/ml) only addition), A': addition of GSSSG and HGF (1 ng/ml), A: addition of GSSSG, B': addition of LA-SSS and HGF (1 ng/ml), B: addition of LA-SSS, PC: positive control (HGF (3 ng/ml) only addition).

In the HGF (1 ng/ml) addition group (PC'), the percentage of BrdU-positive cells, which is an index showing the muscle stem cell activation effect of HGF, significantly increased (p < 0.05 vs. NC), and this value was significantly lower than that of the HGF (3 ng/ml) addition group (PC). This result is consistent with the results shown in Fig. 3 of Ryuichi Tatsumi, et al., "Release of hepatocyte growth factor from mechanically stretched skeletal muscle satellite cells and role of pH and nitric oxide.", Molecular Biology of the Cell, 13(8): 2909-2918 (2002), and reconfirmed that HGF at a concentration of 1 ng/ml cannot sufficiently activate satellite cells.

In the GSSSG 1:8000 molar ratio addition group (A'), the percentage of BrdU-positive cells, which is an index showing the muscle stem cell activation effect of HGF, increased. In the LA-SSS 1:8000 molar ratio addition group (B'), the percentage of BrdU-positive cells, which is an index showing the muscle stem cell activation effect of HGF, significantly increased (p < 0.05 vs. NC), and this value was comparable to that of the HGF (3 ng/ml) addition group (PC). Therefore, it was confirmed that LA-SSS further enhances the muscle stem cell activation effect of HGF.

These results in the cell culture system corresponded to the enhancement effect of GSSSG and LA-SSS on HGF-c-Met affinity shown in Fig. 1. It was suggested that by adding trisulfide compounds such as GSSSG and LA-SSS, muscle hepatocytes can be more easily activated even at low concentrations of HGF. In particular, it was found that LA-SSS has a superior effect on HGF.

## Claims

1. An enhancer of physiological activity of hepatocyte growth factor, comprising a trisulfide compound,
wherein the trisulfide compound is at least one selected from the group consisting of:
glutathione trisulfide and a pharmaceutically acceptable salt thereof;
a compound represented by Formula (1): 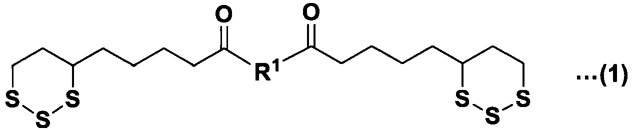
wherein R¹ is a group represented by Formula (2): 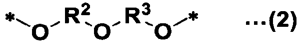
wherein R² and R³ are each independently a C₁₋₄ alkylene group, and * is a bond;
a group represented by Formula (3): 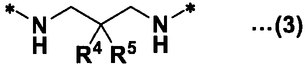
wherein R⁴ and R⁵ are each independently a hydrogen atom or a C₁₋₄ alkyl group optionally having a substituent, R⁴ and R⁵ may form a ring together with the atom bonded thereto, and * is a bond;
a group represented by Formula (4): 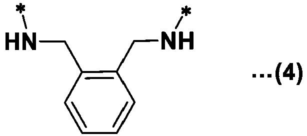
wherein * is a bond; or
a group represented by Formula (5): 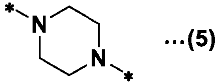
wherein * is a bond, a pharmaceutically acceptable salt thereof, and a cyclodextrin clathrate thereof; and
a compound represented by Formula (6): 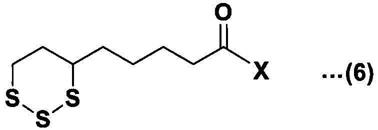
wherein X is -OR⁶ or -NR⁷R⁸,
wherein R⁶, R⁷, and R⁸ are each independently a hydrogen atom; a C₁₋₆ alkyl group optionally having at least one substituent selected from the group consisting of a carboxy group, -OR⁹, -NR¹⁰R¹¹, and - N⁺R¹²R¹³R¹⁴; or -(CH₂CH₂O)ₙR¹⁵,
wherein R⁹, R¹⁰, R¹¹, R¹², R¹³, R¹⁴, and R¹⁵ are each independently a hydrogen atom or a C₁₋₃ alkyl group, and n is an integer of 2 to 5, a pharmaceutically acceptable salt thereof, and a cyclodextrin clathrate thereof.

2. The enhancer of physiological activity according to claim 1, further comprising hepatocyte growth factor.

3. An enhancer of physiological activity of hepatocyte growth factor, which is administered in combination with hepatocyte growth factor, comprising a trisulfide compound,
wherein the trisulfide compound is at least one selected from the group consisting of:
glutathione trisulfide and a pharmaceutically acceptable salt thereof;
a compound represented by Formula (1): 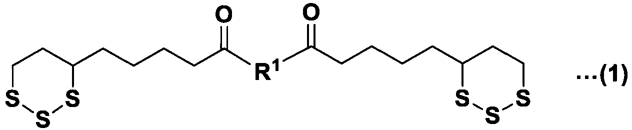
wherein R¹ is a group represented by Formula (2): 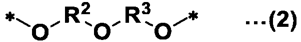
wherein R² and R³ are each independently a C₁₋₄ alkylene group, and * is a bond;
a group represented by Formula (3): 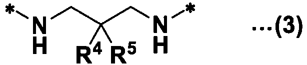
wherein R⁴ and R⁵ are each independently a hydrogen atom or a C₁₋₄ alkyl group optionally having a substituent, R⁴ and R⁵ may form a ring together with the atom bonded thereto, and * is a bond;
a group represented by Formula (4): 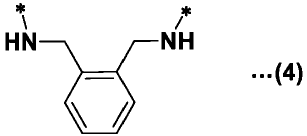
wherein * is a bond; or
a group represented by Formula (5): 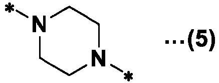
wherein * is a bond, a pharmaceutically acceptable thereof, and a cyclodextrin clathrate thereof; and
a compound represented by Formula (6): 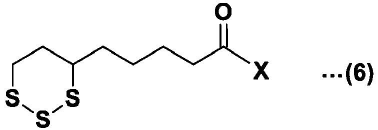
wherein X is -OR⁶ or -NR⁷R⁸,
wherein R⁶, R⁷, and R⁸ are each independently a hydrogen atom; a C₁₋₆ alkyl group optionally having at least one substituent selected from the group consisting of a carboxy group, -OR⁹, -NR¹⁰R¹¹, and - N⁺R¹²R¹³R¹⁴; or -(CH₂CH₂O)ₙR¹⁵,
wherein R⁹, R¹⁰, R¹¹, R¹², R¹³, R¹⁴, and R¹⁵ are each independently a hydrogen atom or a C₁₋₃ alkyl group, and n is an integer of 2 to 5, a pharmaceutically acceptable salt thereof, and a cyclodextrin clathrate thereof.

4. An enhancer of physiological activity of hepatocyte growth factor, which is administered in combination with a trisulfide compound, comprising hepatocyte growth factor,
wherein the trisulfide compound is at least one selected from the group consisting of:
glutathione trisulfide and a pharmaceutically acceptable salt thereof;
a compound represented by Formula (1): 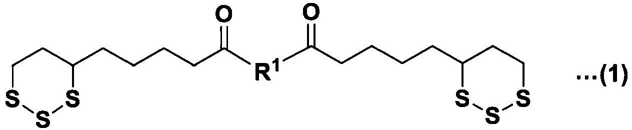
wherein R¹ is a group represented by Formula (2): 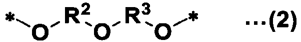
wherein R² and R³ are each independently a C₁₋₄ alkylene group, and * is a bond;
a group represented by Formula (3): 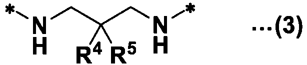
wherein R⁴ and R⁵ are each independently a hydrogen atom or a C₄ alkyl group optionally having a substituent, R⁴ and R⁵ may form a ring together with the atom bonded thereto, and * is a bond;
a group represented by Formula (4): 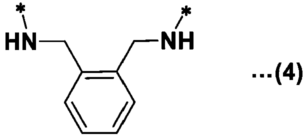
wherein * is a bond; or
a group represented by Formula (5): 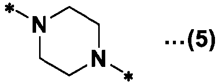
wherein * is a bond, a pharmaceutically acceptable salt thereof, and a cyclodextrin clathrate thereof; and
a compound represented by Formula (6): 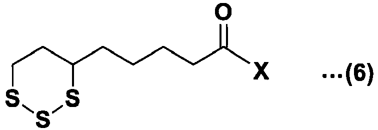
wherein X is -OR⁶ or -NR⁷R⁸,
wherein R⁶, R⁷, and R⁸ are each independently a hydrogen atom; a C₁₋₆ alkyl group optionally having at least one substituent selected from the group consisting of a carboxy group, -OR⁹, -NR¹⁰R¹¹, and - N⁺R¹²R¹³R¹⁴; or -(CH₂CH₂O)ₙR¹⁵,
wherein R⁹, R¹⁰, R¹¹, R¹², R¹³, R¹⁴, and R¹⁵ are each independently a hydrogen atom or a C₁₋₃ alkyl group, and n is an integer of 2 to 5, a pharmaceutically acceptable salt thereof, and a cyclodextrin clathrate thereof.

5. The enhancer of physiological activity according to claim 3, wherein the trisulfide compound and hepatocyte growth factor are administered simultaneously or separately.

6. The enhancer of physiological activity according to claim 1, which is an enhancer for the binding between hepatocyte growth factor and c-Met.

7. The enhancer of physiological activity according to any one of claims 1 to 6, wherein the trisulfide compound is lipoic acid trisulfide or a pharmaceutically acceptable salt thereof.

8. The enhancer of physiological activity according to any one of claims 1 to 6, wherein the trisulfide compound is glutathione trisulfide or a pharmaceutically acceptable salt thereof.
